# EUROPÄISCHE PATENTSCHRIFT

(11) **EP 0 792 127 B1**
(45) Veröffentlichungstag und Bekanntmachung des Hinweises auf die Patenterteilung: **02.01.2002**
(21) Anmeldenummer: 95936961.2
(22) Anmeldetag: 20.11.1995
(51) Int. Cl.: A61F 2/36, A61B 17/74, A61F 2/46

(54) **GELENKPROTHESE**
JOINT PROSTHESIS
PROTHESE D'ARTICULATION

(30) Priorität: 19.11.1994 DE 4442206
(43) Veröffentlichungstag der Anmeldung: 03.09.1997
(73) Patentinhaber: Biomet Merck Deutschland GmbH, 14167 Berlin (DE)
(72) Erfinder: LOB, Günter, D-81377 München (DE); FISCHER, Hans-Joachim, D-12277 Berlin (DE); STEÜR, Gerd, D-10551 Berlin (DE); KRANZ, Curt, D-10825 Berlin (DE)
(74) Vertreter: Maikowski, Michael, Dipl.-Ing. Dr.
(86) Internationale Anmeldenummer: DE9501642
(87) Internationale Veröffentlichungsnummer: WO9615737

(56) Entgegenhaltungen:
- EP-A- 0 010 527
- EP-A- 0 399 920
- EP-A- 0 586 824
- WO-A-85/05027
- DE-A- 2 854 334
- DE-U- 8 701 164
- DE-U- 9 418 963
- FR-A- 1 099 519
- FR-A- 2 183 230
- FR-A- 2 575 383
- FR-A- 2 629 707
- FR-A- 2 639 820
- FR-A- 2 651 118
- FR-A- 2 674 119

## Beschreibung

Die Erfindung betrifft eine modulare Gelenkprothese der im Oberbegriff des Anspruches 1 angegebenen Art.

Derartige Schaftprothesen werden in unterschiedlichen Formen und Größen hergestellt, um eine optimale Versorgung des jeweiligen Patienten sicherzustellen.

Aus der internationalen Patentanmeldung WO 85/05027 ist ein künstliches Gelenksystem, insbesondere eine Hüftgelenkprothese zur zementlosen Implantation bekannt, welche einen im Oberschenkel des Patienten einbringbaren Schaftteil und ein mit diesem Schaftteil verbundenes Gelenkanschlußelement aufweist. In dem proximalen Bereich des Schaftteils ist eine Gewindehülse vorgesehen, in welche das Gelenkanschlußelement eingeschraubt wird, wobei die Längsachse des Schaftteils und die Längsachse des Gelenkanschlußelements gegeneinander geneigt sind. Das Gelenkanschlußelement weist dementsprechend einen Gewindezapfen auf, welcher an seinem proximalen Ende miteinem Konusschaftteil zur Aufnahme der Gelenkkugel verbunden ist. Der Einschraubvorgang ist abgeschlossen, wenn der Konusschaftteil des Gelenkanschlußelements fest an dem zur Implantation vorbereiteten proximalen Bereich des Oberschenkelknochens anliegt.

Darüber hinaus ist aus dem deutschen Gebrauchsmuster DE 87 01 164 U1 eine Gelenkprothese mit einem in einem Röhrenknochen einsetzbaren Schaftteil und einem sich daran anschließenden Kopfteil mit einem Gelenkanschlußelement bekannt. Dabei schließen die Längsachsen von Schaft und Gelenkanschlußelement einen stumpfen Winkel, den Inversionswinkel, ein. Das Kopfteil weist eine im wesentlichen unter dem Inversionswinkel zur Schaftachse geneigt angeordnete Durchgangsbohrung zur Aufnahme des Schaftes des Gelenkanschlußteils auf, welche dem Querschnitt des Schaftes des mit dem Kopfteil zu verbindenden Gelenkanschlußelements angepaßt ist.

Die vorstehend beschriebenen Lösungen weisen jedoch den Nachteil auf, daß bei Reoperationen einerseits lediglich ein vollständiger Ersatz des Gelenks einschließlich Gelenkkugel erfolgen kann und andererseits ein Lösen des Kopfteils von dem Schaftteil bei einem festsitzenden Konus wegen des in dem Kopfteil eingesetzten Gelenkanschlußelement nur mit großem Aufwand - und dadurch mit der Gefahr einer Lockerung des Schaftteils der Gelenkprothese verbunden - möglich ist.

Ausgehend von den Mängeln des Standes der Technik liegt der Erfindung die Aufgabe zugrunde, einen modularen Hüftprothesenschaft der eingangs genannten Gattung zu schaffen, dessen konstruktiver Aufbau auf besonders einfache Weise auch ein Auswechseln von Teilelementen im Gelenkbereich gestattet, um eine Anpassung an unterschiedlich fortgeschrittene Krankheitszustände zu ermöglichen.

Diese Aufgabe wird mit den Merkmalen des Anspruches 1 gelöst.

Die Erfindung schließt die Erkenntnis ein, daß zur Erhaltung der natürlichen Gelenkkugel bei einer modularen Prothese ein zusätzliches, alternativ anwendbares Kopfteil günstig ist, welches mit einer Knochenschraube in Richtung des Halses der Gelenkkugel versehen ist, die in die separierte natürliche Gelenkkugel einschraubbar ist. Damit kann das Gelenk mit seinen natürlichen Teilen Gelenkkugel/Pfanne gegebenenfalls noch längere Zeit erhalten bleiben. Das Kopfteil kann dann später gegebenenfalls nahezu ohne Belastung für die Knochensubstanz des Patienten und ohne Auslockerung des Sitzes des Prothesenschaftes mit geringem Aufwand ausgetauscht werden.

Mit den erfindungsgemäßen Maßnahmen kann die dargestellte Prothese zunächst auch eine herkömmliche Hüftschraube ersetzen, wie sie im Falle von Frakturen im Femurbereich häufig eingesetzt werden. Eine spätere Reoperation ist dann insoweit vereinfacht, als nur das Kopfteil der modularen Schaftprothese gegen ein solches mit einer künstlichen Gelenkkugel ausgetauscht werden muß, um erneut eine Vollprothese zu erhalten.

Die erfindungsgemäße modular ausgebildete Hüftprothese weist als Gelenkanschlußelement und Schaftteil ausgebildete Einzelelemente auf, welche vorzugsweise durch Zusammenfügen ihrer proximalen bzw. distalen Enden miteinander verbunden werden. Das Gelenkanschlußelement dient der Herstellung einer sicheren Verbindung zwischen dem Prothesenschaft und dem Hüftgelenk.

Durch die Modularität der Prothese kann bei der Anpassung des modularen Hüftgelenkschaftes an die jeweiligen anatomischen Bedingungen das Gelenkanschlußelement auch ohne besonderen Krafteinsatz in seiner Position verändert und danach auf einfache Weise erneut sicher befestigt werden.

Unter Erhalt des natürlichen Gelenkkopfes ist mit den erfindungsgemäßen Maßnahmen in vorteilhafter Weise eine Korrektur der Position des Gelenkanschlußelements zwecks Anpassung an die besonderen anatomischen Bedingungen des Patienten (beispielsweise eine relativ geringe axiale Verschiebung in Halsrichtung) während des Implantationsvorgangs möglich.

Vorteilhaft ist auch, daß bei der erfindungsgemäßen Prothese die Verbindungen der modularen Elemente untereinander auch nach Implantationsdauer anläßlich einer Nachoperation mit geringem Aufwand gelöst werden können, wenn dies aus medizinischer Sicht, beispielsweise zwecks Austausch oder Neueinstellung des Gelenkanschlußelements, erforderlich ist. Herkömmliche Verbindungen haften dagegen häufig derart fest zusammen, daß bei einer notwendigen Demontage zum Nachteil des Patienten Beschädigungen des Oberschenkelknochens oder zumindest eine unerwünschte Lockerung des Endteils häufig nicht vermieden werden können.

Entsprechend der bevorzugten Ausführungsform der Erfindung ist - um zwecks weiterer Vereinfachung der Anpassung einer bereits implantierten Prothese an geänderte anatomische Bedingungen des Patienten eine Trennung des das Gelenkanschlußelement tragende Kopfteil von dem im Oberschenkelknochen befindlichen Endteil vornehmen zu können, ohne das Endteil einer zusätzlichen, den festen Sitz des Implantats beeinträchtigenden mechanischen Belastung zu unterwerfen - der zylindrische Kanal im Kopfteil der modularen Gelenkprothese als Gewindebohrung ausgebildet. Der Durchmesser dieser Gewindebohrung ist größer als der Durchmesser der den Zuganker aufnehmenden Gewindebohrung im Endteil.

Ein Durchmesser-Verhältnis im Bereich von 1,5 bis 2,5 sichert, daß auf dem proximal vorgesehenen, kegelstumpfförmigen Ende des Endteils ein im wesentlichen kreisringförmiger Abschnitt der Deckfläche des Kegelstumpfes als Widerlager für einen Schraubbolzen zur Verfügung steht, welcher nach Entfernen des Zugankers in das Kopfteil geschraubt werden kann. Der in das Kopfteil eingeschraubte Bolzen stützt sich auf dem Widerlager ab und erzeugt bei weiterer Schraubbewegung eine axiale Druckkraft, welche die (trotz fehlendem Zuganker) feste konische Steckverbindung zwischen Kopf- und Endteil soweit löst, daß das Kopfteil zwecks Austausch entnommen oder bezüglich des Endteils in gewünschtem Maße auf dessem am proximalen Ende vorgesehenen, kegelstumpfförmigen Zapfen geschwenkt werden kann.

Nach einer anderen vorteilhaften Ausführung der Erfindung erstreckt sich das für den Schraubbolzen vorgesehene Gewinde im Kopfteil der modularen Gelenkprothese über die gesamte Länge des vorhandenen zylindrischen Kanals. Sowohl für die Gewindebohrung im Kopfteil des Hüftprothesenschaftes als auch für den Gewindeabschnitt am proximalen Ende des Endteils ist ein metrisches Gewinde vorgesehen. Dabei weist das Gewinde der Gewindebohrung im Kopfteil eine geringere Steigung auf als das Gewinde am proximalen Ende des Endteils und erleichtert dadurch das Erzeugen der zum Lösen der festen konischen Steckverbinduung erforderlichen Druckkraft durch den Schraubbolzen.

Entsprechend einer günstigen Weiterbildung der Erfindung weist das proximale Ende der im Endteil vorgesehenen Gewindebohrung eine Ausnehmung auf, welche in günstiger Weise als symmetrisch zur Längsachse der Bohrung angeordnete Anfasung ausgebildet ist. Dadurch wird in vorteilhafter Weise erreicht, daß der zum Lösen der konischen Steckverbindung verwendete Schraubbolzen, dessen freies Ende ebenfalls mit gleichem Winkel gefast ausgebildet ist, beim Abstützen auf dem proximalen Ende des Endteils nicht mit dem Gewinde der in diesem Bereich vorgesehenen Gewindebohrung in Wirkkontakt gelangen kann. Dadurch wird beim Erzeugen der zum Lösen der konischen Steckverbindung zwischen Kopf- und Schaftteil erforderlichen Druckkraft eine Deformation der ersten Gewindegänge vermieden. Ein deformiertes Gewinde würde ein erneutes Verschrauben des Zugankers in das proximale Ende des Endteils nach Anpassen oder Austausch des Kopfteils unmöglich machen.

Entsprechend der bevorzugten Ausführungsform der Erfindung ist das Gelenkanschlußelement als ein in einen geneigt zur Längsachse des Schaftteils an dessen proximalen Ende angeordneten Durchgangskanal einschiebbares und dort durch Haltemittel in verschiedenen Positionen fixierbares Teil ausgebildet. Auf diese Weise kann zusätzlich Variabilität durch die Modularbauweise des Schaftes auch im Halsbereich eine Feinabstimmung an die individuellen Gegebenheiten des Patienten vorgenommen werden.

Die Haltemittel sind derart ausgebildet, daß sie eine radiale Expansion des Querschnitts des in den Schaftteil eingeschobenen Abschnitts des Gelenkanschlußelements als Spreizmittel zur Verklemmung ermöglichen und somit ohne besonderen Kraftaufwand aktivierbar sind.

Nach einer anderen günstigen Weiterbildung der Erfindung ist als Durchgangskanal eine zylindrische Bohrung vorgesehen. Der in die Durchgangsbohrung eingeschobene Bereich des Schaftes des Gelenkanschlußelements weist die Form einer in Richtung der Längsachse geschlitzten Hülse auf, so daß durch Einschrauben eines konischen Gewindebolzens ein Spreizen der Hülse erreichbar ist und damit eine sichere form- und kraftschlüssige Verbindung zwischen Schaftteil und Gelenkanschlußelement entsteht.

Durch Verringern der Spreizung ist die Verbindung zwischen Schaftteil und Gelenkanschlußelement in günstiger Weise lösbar und eine Auswechselbarkeit bzw. Lagekorrektur des Gelenkanschlußmittels möglich. Dies sichert eine vereinfachte Anpassung der Gelenkprothese an die jeweiligen patientenspezifischen anatomischen Bedingungen. Ein nachträglich erforderlicher Austausch des Befestigungsmittels kann dabei erfolgen, ohne daß eine besondere mechanische Belastung des im Oberschenkelknochen befindlichen Schaftteils des Hüftprothesenschaftes erfolgt, welche im ungünstigsten Fall zu einer irreversiblen Lockerung des Schaftteils führen kann.

Entsprechend einer vorteilhaften Ausführungsform der Erfindung ist das Gelenkanschlußelement als Knochenschraube ausgebildet, welche dann zum Einsatz gelangt, wenn der Femur geschädigt ist, aber die natürliche Gelenkkugel erhaltenswert ist.

Schreitet die Schädigung der Gelenkkugel weiter voran, so wird nach einer Weiterbildung der Erfindung anstelle der Knochenschraube ein Kugelkopf mit einem Zapfen als Gelenkanschlußelement benutzt, wobei das freie Ende des zapfens als geschlitzte Hülse ausgebildet ist. Dieser Austausch der Knochenschraube, bei dem der Kugelkopf in die vorhandene Hüftgelenkpfanne eingesetzt wird, erfolgt bei geringster mechanischer Belastung des bereits implantierten Schaftteils ohne Gefahr für dessen Sitz.

Entsprechend einer anderen Weiterbildung der Erfindung ist der Schaftteil in ein die künstliche Gelenkkugel tragenden Kopfteil und ein Halsteil trennbar, wobei die beiden Teilelemente mittels einer konischen Steckverbindung zusamengefügt sind.

Entsprechende konische Steckverbindung finden auch bei den Teilelementen des modulartig zusammengesetzten Schafts Anwendung, wobei dieser in Längsrichtung durch einen Zuganker oder eine Schraubverbindung gesichert bzw. gesetzt werden kann, welcher - durch einen zusätzlichen axialen Kanal im Kopfteil geführt - in eine auf gleicher Achse liegende Gewindebohrung im Endteil des Schaftes einschraubbar ist.

Um zwecks weiterer Vereinfachung der Anpassung einer bereits implantierten Prothese an geänderte anatomische Bedingungen des Patienten eine Trennung des das Gelenkanschlußelement tragenden Kopfteils von dem im Oberschenkelknochen befindlichen Endteil vornehmen zu können, ohne das Endteil einer zusätzlichen, den festen Sitz des Implantats beeinträchtigenden mechanischen Belastung zu unterwerfen, ist entsprechend einer günstigen Weiterbildung der Erfindung der zylindrische Kanal im Kopfteil der modularen Gelenkprothese als Gewindebohrung ausgebildet. Der Durchmesser dieser Gewindebohrung ist größer als der Durchmesser der den Zuganker aufnehmenden Gewindebohrung im Endteil.

Ein Durchmesser-Verhältnis im Bereich von 1,5 bis 2,5 sichert, daß auf dem proximal vorgesehenen, kegelstumpfförmigen Ende des Endteils ein im wesentlichen kreisringförmiger Abschnitt der Deckfläche des Kegelstumpfes als Widerlager für einen Schraubbolzen zur Verfügung steht, welcher nach Entfernen des Zugankers in das Kopfteil geschraubt werden kann. Der in das Kopfteil eingeschraubte Bolzen stützt sich auf dem Widerlager ab und erzeugt bei weiterer Schraubbewegung eine axiale Druckkraft, welche die (trotz fehlendem Zuganker) feste konische Steckverbindung zwischen Kopf-und Endteil soweit löst, daß das Kopfteil zwecks Austausch entnommen oder bezüglich des Endteils in gewünschtem Maße auf dessem am proximalen Ende vorgesehenen, kegelstumpfförmigen Zapfen geschwenkt werden kann.

Nach einer anderen vorteilhaften Ausführung der Erfindung erstreckt sich das für den Schraubbolzen vorgesehene Gewinde im Kopfteil der modularen Gelenkprothese über die gesamte Länge des vorhandenen zylindrischen Kanals. Sowohl für die Gewindebohrung im Kopfteil des Hüftprothesenschaftes als auch für den Gewindeabschnitt am proximalen Ende des Endteils ist ein metrisches Gewinde vorgesehen. Dabei weist das Gewinde der Gewindebohrung im Kopfteil eine geringere Steigung auf als das Gewinde am proximalen Ende des Endteils und erleichtert dadurch das Erzeugen der zum Lösen der festen konischen Steckverbinduung erforderlichen Druckkraft durch den Schraubbolzen.

Entsprechend einer anderen Weiterbildung der Erfindung weist das proximale Ende der im Endteil vorgesehenen Gewindebohrung eine Ausnehmung auf, welche in günstiger Weise als symmetrisch zur Längsachse der Bohrung angeordnete Anfasung ausgebildet ist. Dadurch wird in vorteilhafter Weise erreicht, daß der zum Lösen der konischen Steckverbindung verwendete Schraubbolzen, dessen freies Ende ebenfalls mit gleichem Winkel gefast ausgebildet ist, beim Abstützen auf dem proximalen Ende des Endteils nicht mit dem Gewinde der in diesem Bereich vorgesehenen Gewindebohrung in Wirkkontakt gelangen kann. Dadurch wird beim Erzeugen der zum Lösen der konischen Steckverbindung zwischen Kopf- und Schaftteil erforderlichen Druckkraft eine Deformation der ersten Gewindegänge vermieden. Ein deformiertes Gewinde würde ein erneutes Verschrauben des Zugankers in das proximale Ende des Endteils nach Anpassen oder Austausch des Kopfteils unmöglich machen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1 einen Teil-Längsschnitt durch eine bevorzugte Ausführungsform der Erfindung,
- Figur 2 die schematisierte Darstellung eines in Figur 1 gezeigten Befestigungsmittels in Seitenansicht mit Teil-Längsschnitt,
- Figur 3 eine günstige Weiterbildung des in Figur 1 gezeigten Befestigungsmittels in Seitenansicht mit Teil-Längsschnitt, sowie
- Figur 4 die Einzelheit E gemäß Figur 1 in vergrößerter Darstellung.

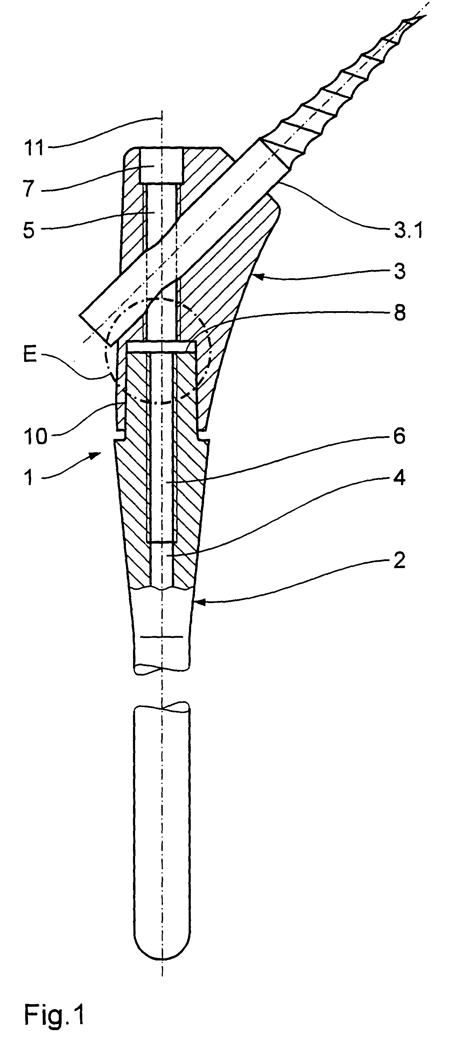

Der in Figur 1 dargestellte modulare Gelenkprothese 1 besteht aus einem Schaftteil 2 und einem Kopfteil 3, welche jeweils eine Profilierung in Längsrichtung aufweisen. Das Kopfteil 3 trägt das als Knochenschraube ausgebildete Gelenkanschlußmittel 3.1, durch welches die Verbindung der Gelenkprothese 1 mit dem Hüftgelenk herstellbar ist. Die Längsachsen des Gelenkanschlußelements 3.1 und des Prothesenschafts 1 schließen einen Winkel von etwa 135° ein. Die Verbindung zwischen Kopfteil 3 und Gelenkanschlußelement 3.1 erfolgt sowohl form- als auch kraftschlüssig.

Die Teilelemente 2 und 3 des Schaftes 1 sind mittels einer konischen Steckverbindung 10 auf gleicher Achse liegend und gegeneinander verschwenkbar verbunden. Zur Sicherung dieser Steckverbindung gegen axiales Lösen ist ein (nicht dargestellter) Zuganker vorgesehen, welcher durch den als Gewindebohrung ausgebildeten Kanal 5 des Kopfteils 3 geführt und in den als Gewindebohrung 6 ausgebildeten proximalen Bereich des im Schaftteil 2 vorgesehenen Kanals 4 geschraubt wird. Das Einschrauben erfolgt soweit, daß sich das proximale Ende des Zugankers in der Ausnehmung 7 abstützt und dabei die Einzelteile 2 und 3 der modularen Gelenkprothese 1 axial gegeneinander bewegt werden, bis die konische Steckverbindung 10 die gewünschte Festigkeit aufweist.

Um die Position des Kopfteils 3 mit dem Gelenkanschlußelement 3.1 in Bezug auf das Schaftteil 2 durch Schwenken ändern zu können oder das Kopfteil 3 auszutauschen, muß die konische Steckverbindung 10 erneut gelöst werden. Nach Lösen und Herausdrehen des (nicht dargestellten) Zugankers wird ein (ebenfalls nicht dargestellter) Schraubbolzen in die Gewindebohrung 5 des Kopfteils 3 eingeschraubt. Da der Durchmesser D₁ der Gewindebohrung 5 im Kopfteil 3 einen größeren Wert aufweist als der Durchmesser D₂ des proximalen, mit einem Gewinde versehenen Abschnitt 6 der zentralen Bohrung 4 des Schaftteils 2, stützt sich der Schraubbolzen auf einem sich in seinem Flächenmaß durch die Durchmesser-Differenz D₁ - D₂ festgelegten Kreisring auf der Deckfläche 8 des als Kegelstumpf ausgebildeten proximalen Endes des Kopfteils 3 und ist somit in der Lage, eine axial gerichtete Druckkraft zu erzeugen. Diese Kraft löst die durch die Wirkung des Zugankers sehr feste konische Steckverbindung 10 und ermöglicht auf bequeme Art und Weise eine erneute Anpassung der Gelenkprothese 1 an veränderte körperliche Bedingungen eines Patienten.

In diesem Zusammenhang hat es sich für die Handhabbarkeit der Mittel zum Erzeugen der zum Lösen der konischen Steckverbindung erforderlichen Druckkraft als günstig erwiesen, ein Durchmesser-Verhältnis der Bohrungen 4,5 im Bereich von 1,5 bis 2,5 vorzusehen und die Bohrung 5 über die gesamte Länge mit einem Gewinde zu versehen. Es werden metrische Gewinde verwendet, wobei das Gewinde der Gewindebohrung 5 im Kopfteil 3 eine geringere Steigung aufweist als das Gewinde im proximalen Abschnitt 6 der Bohrung 4 des Schaftteils 2.

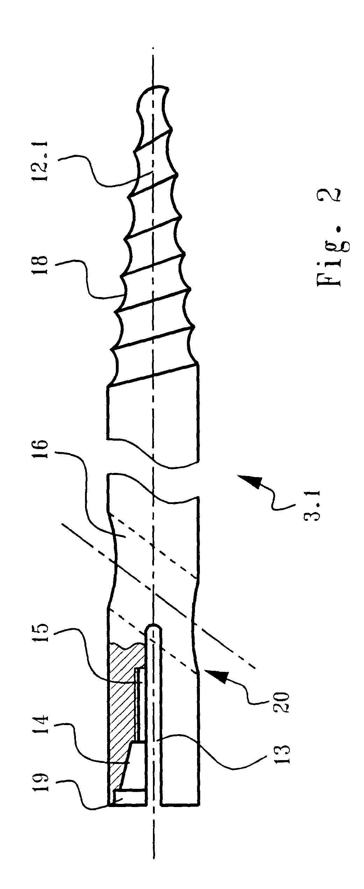

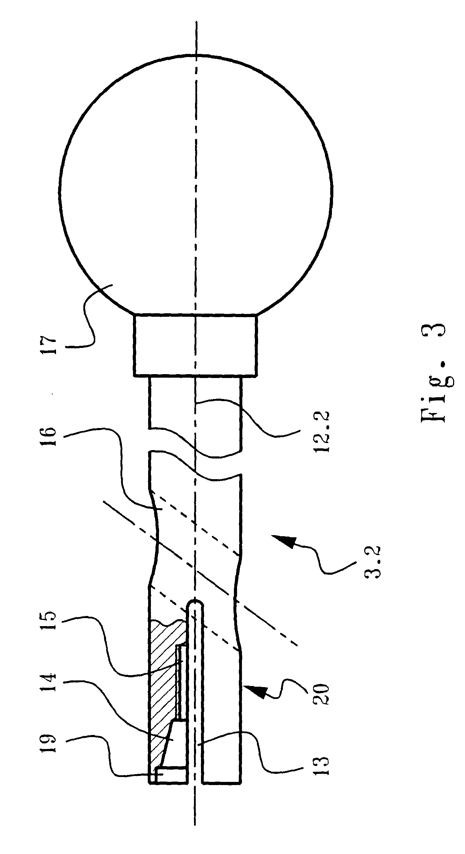

In den Figuren 2 und 3 sind vorteilhafte Ausführungsformen von Gelenkanschlußelementen 3.1, 3.2 als Teilschnitt schematisiert dargestellt. Sowohl das dem Gewinde 18 abgewandte hülsenförmige Ende der Knochenschraube 3.1 als auch der zylindrische Hohlzapfen des einen Kugel-Kopf 17 aufweisenden Gelenkanschlußelements 3.2 sind mit einem Schlitz 13 versehen. Die Wandungen des Schlitzes 13 sind durch einen (nicht dargestellten) Bolzen, der sich - in die Gewindebohrung 15 eingeschraubt - an der Wandung der konischen Ausnehmung 14 abstützt, abspreizbar, so daß sich eine Durchmesservergrößerung ergibt. Dadurch ist ein fester Sitz des Gelenkanschlußelements im Kopfteil 3 gesichert. Die in Bezug auf die Längsachsen 12.1 und 12.2 der Gelenkanschlußelemente 3.1 und 3.2 geneigt angeordnete Bohrung 16 ermöglicht die Bedienung des (nicht dargestellten) Zugankers bzw. des (nicht dargestellten) Schraubbolzens zum Verbinden bzw. Trennen der Einzelteile 2 und 3 der modular aufgebauten Gelenkprothese 1.

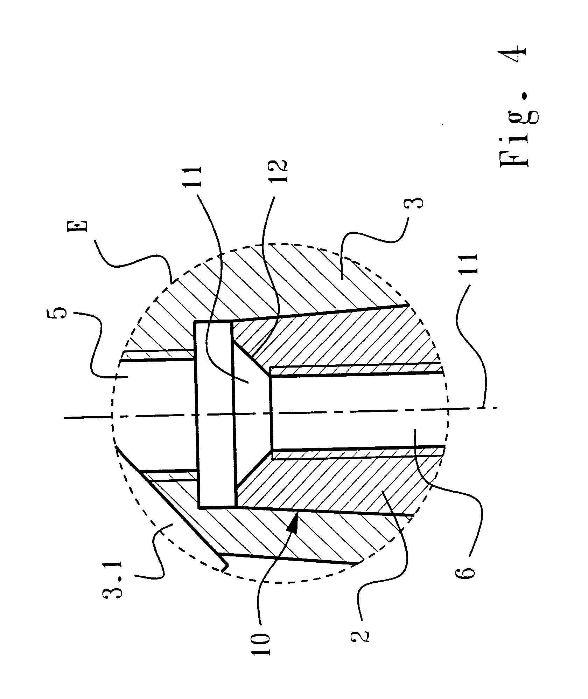

Die in Figur 4 vergrößert dargestellte Einzelheit E gemäß Figur 1 zeigt eine vorteilhafte Weiterbildung der Erfindung. Um zu verhindern, daß durch den Wirkungseingriff des (nicht dargestellten) Schraubbolzens an der Oberseite des proximalen Endes des Endteils 2 eine Beschädigung des dort vorgesehenen Gewindeabschnitts 6 möglich wird, ist an dem proximalen Ende der Gewindebohrung eine als Anfasung ausgebildete Ausnehmung 11 vorgesehen. Die Neigung der Flanken 12 beträgt 45° in Bezug auf die Längsachse 11 und entspricht der Flankenneigung der Fasung am Gewindeende des (nicht dargestellten) Schraubbolzens. Dadurch kann ein optimaler Krafteintrag in das Endteil 2 erfolgen, um ein Lösen der konischen Steckverbindung 10 zwischen Endteil 2 und Kopfteil 3 mit relativ geringem Krafteinsatz zu ermöglichen. Eine Beschädigung des Gewindes des Bohrungsabschnitts 6 würde eine erneute Befestigung des Kopfteils mit axialer Sicherung der konischen Steckverbindung mittels des Zugankers unmöglich machen. Es ist ersichtlich, daß bei weiteren (in der Zeichnung nicht dargestellten) Ausführungsformen durch die erzielte Modularität in einem System von Schaftprothesen zur Erreichung unterschiedlicher individuell bemessener Prothesenformen Elemente verschiedener Größen kombinierbar sind.

Die erfindungsgemäße Stufung der Gewindedurchmesser kann ebenfalls zwischen jeweils unterschiedlichen der aufeinanderfolgenden Elementen vorgesehen sein. Damit ist es beispielsweise möglich eine Trennung - wenn gewünscht - nicht nur zwischen dem Kopfteil und dem in Richtung distal nachfolgenden Schaftteil, sondern auch zwischen Schaftteilen untereinander, zu bewirken. Für ein gezieltes "Anwählen" einer Verbindung zum Lösen müßte dann bei sich zum Kopfende hin stufenweise vergrößernden Gewindedurchmessern ein Bolzen ausgesucht werden, dessen Außengewinde mit dem Innengewinde in Eingriff kommt, welches sich in dem auf der proximalen Seite der beabsichtigten Trennstelle gelegenen Schaftelements befindet. Er stützt sich dann mit dem Rand seines Endes auf dem Rand der Bohrung des distal benachbarten Elements ab und trennt die ausgewählte Verbindung.

Bei einem modularen System mit unterschiedlich gekrümmten Schaftelementen lassen sich individuell geformte Schäfte durch unterschiedliche relative Winkelpositionierung erzeugen, ohne daß die Lösbarkeit im vorstehenden Sinne irgendwie eingeschränkt wäre.

## Patentansprüche

1. Gelenkprothese (1) mit einem in einem Röhrenknochen einsetzbaren, eine Längsbohrung (4) aufweisenden Schaftteil (2) und einem sich daran proximal anschließenden Kopfteil (3) mit einem in diesem längsverschieblich gelagerten Gelenkanschlußelement (3.1, 3.2), wobei die Längsachsen von Schaftteil (2) und Gelenkanschlußelement (3.1, 3.2) einen stumpfen Winkel, den Inversionswinkel, einschließen, und das Kopfteil (3)einen Hülsenbereich aufweist, der eine im wesentlichen unter dem Inversionswinkel zur Schaftachse (11) geneigte Durchgangsbohrung aufweist, die dem Querschnitt des Schafts (20) des in diese Bohrung einfügbaren Gelenkanschlußelements (3.1, 3.2) angepaßt ist, **dadurch gekennzeichnet, daß** das Kopfteil (3) eine axiale, in Richtung des Schaftteils (2) verlaufende Bohrung (5) aufweist, welche das in dem Kopfteil (3) gelagerte Gelenkanschlußelement (3.1, 3.2) durchquert.

2. Gelenkprothese nach Anspruch 1 **dadurch gekennzeichnet, daß** die Bohrung (5) mit einem Gewinde für ein zum Lösen der Steckverbindung mit dem jeweils nachfolgenden Schaftteil dienendes Schraubmittel vorgesehen ist, wobei der Durchmesser (D₁) der Bohrung (5) größer ist als der Durchmesser (D₂) einer in dem nachfolgenden Schaftteil angeordneten Bohrung (4).

3. Gelenkprothese nach Anspruch 1 oder 2, **dadurch gekennzeichnet, daß** das Verhältnis des Durchmessers (D₁) der Bohrung (5) im Kopfteil (3) zum Durchmesser (D₂) der Bohrung im nachfolgenden Schaftteil einen Wert im Bereich von 1,5 bis 2,5 aufweist.

4. Gelenkprothese nach Anspruch 2 oder 3, **dadurch gekennzeichnet, daß** sich das Gewinde in der im Kopfteil (3) bzw. in dem in Richtung proximal vorangehenden Schaftteil (2) angeordneten Bohrung (5) über deren gesamte Länge erstreckt.

5. Gelenkprothese nach einem der vorhergehenden Ansprüche, **dadurch gekennzeichnet, daß** die im nachgeordneten Schaftteil (2) vorgesehene Bohrung (4) nur im Bereich ihres proximalen Endes (6) mit einem Gewinde versehen ist.

6. Gelenkprothese nach Anspruch 5, **dadurch gekennzeichnet, daß** das proximale Ende der Gewindebohrung (6) des nachgeordneten Schaftteils (2) eine Anfasung (11) aufweist.

7. Gelenkprothese nach Anspruch 1 oder 2, **dadurch gekennzeichnet, daß** das Gelenkanschlußelement (3.1, 3.2) mit seinem Schaft (20) in dem hülsenförmigen Bereich des Kopfteils (3) arretierbar gelagert ist.

8. Gelenkprothese nach Anspruch 7, **dadurch gekennzeichnet, daß** der das Kopfteil (3) durchdringende Schaft (20) des Gelenkanschlußelements (3.1, 3.2) durch Spreizmittel zu seiner Arretierung expandierbar ist.

9. Gelenkprothese nach Anspruch 8, **dadurch gekennzeichnet, daß** der Schaft (20) mindestens teilweise als eine spreizfähige, mit in Längsrichtung verlaufenden Schlitzen (13) versehene Hülse ausgebildet ist.

10. Gelenkprothese nach Anspruch 9, **dadurch gekennzeichnet, daß** zum Spreizen des geschlitzten Bereichs des Gelenkanschlußelements (3.1, 3.2) ein Schraubbolzen mit einem sich zu seinem Schaftende hin verjüngenden Bereich vorgesehen ist.

11. Gelenkprothese nach einem der vorhergehenden Ansprüche, **dadurch gekennzeichnet, daß** das Gelenkanschlußelement als Knochenschraube (3.1) oder als Gelenkkugel (3.2) ausgebildet ist.

12. Gelenkprothese nach Anspruch 11, **dadurch gekennzeichnet, daß** die Gelenkkugel mit dem Kopfteil (3) mittels einer konischen Steckverbindung zusammenfügbar ist.

13. Bausatz zur Herstellung einer Gelenkprothese nach Anspruch 1 oder 2, **gekennzeichnet durch** eine Auswahl von Kopfteilen (3) unterschiedlicher Länge und/oder unterschiedlichen Durchmessers sowie eine Auswahl von Schaftteilen (2) unterschiedlicher Länge, unterschiedlichen Durchmessers oder unterschiedlicher Krümmung.

14. Bausatz nach Anspruch 13, **dadurch gekennzeichnet, daß** für die Kopfteile (3) ein Durchmesser im Bereich von 12 bis 17 mm und für die Schaftteile (2) ein Längenbereich von 200 bis 320 mm und ein Durchmesserbereich von 10 bis 14 mm vorgesehen ist

## Claims

1. Joint prosthesis (1) having a shaft part (2) with longitudinal bore (4) insertable in a long bone, and a head part (3) adjoining same on the proximal side and having a joint connecting element (3.1, 3.2) mounted longitudinally displaceable therein, wherein the longitudinal axes of the shaft part (2) and the joint connecting element (3.1, 32.) include an obtuse angle, the inversion angle, and the head part (3) has a sleeve area which has a full-length bore which is inclined substantially with the inversion angle relative to the shaft axis (11) and matches the cross-section of the shaft (20) of the joint connecting element (3.1, 3.2) which is insertable in this bore, **characterised in that** the head part (3) has an axial bore (5) running in the direction of the shaft part (2) and crossing through the joint connecting element (3.1, 3.2) which is mounted in the head part (3).

2. Joint prosthesis according to claim 1 **characterised in that** the bore (5) has a thread for screw means which serve to release the push-fit connection with the following shaft part, wherein the diameter (D₁) of the bore (5) is larger than the diameter (D₂) of a bore (4) mounted in the following shaft part.

3. Joint prosthesis according to claim 1 or 2 **characterised in that** the ratio of the diameter (D₁) of the bore (5) in the head part (3) relative to the diameter (D₂) of the bore in the following shaft part is in the range from 1.5 to 2.5.

4. Joint prosthesis according to claim 2 or 3 **characterised in that** the thread in the bore (5) mounted in the head part (3) or in the preceding shaft part (2) on the proximal side extends over the entire length.

5. Joint prosthesis according to one of the preceding claims **characterised in that** the bore (4) provided in the following shaft part (2) is only provided with a thread in the region of its proximal end (6).

6. Joint prosthesis according to claim 5 **characterised in that** the proximal end of the threaded bore (6) of the following shaft part (2) has a bevelled area (11).

7. Joint prosthesis according to claim 1 or 2 **characterised in that** the joint connecting element (3.1, 3.2) is mounted lockable by its shaft (20) in the sleeve-like area of the head part (3).

8. Joint prosthesis according to claim 7 **characterised in that** the shaft (20) of the joint connecting element (3.1, 3.2) passing through the head part (3) can be expanded by expanding means to produce a locking action.

9. Joint prosthesis according to claim 8 **characterised in that** the shaft (20) is formed at least in part as an expanding sleeve provided with longitudinally aligned slits (13).

10. Joint prosthesis according to claim 9 **characterised in that** in order to spread out the slit area of the joint connecting element (3.1, 3.2) a screw bolt is provided which has an area tapering towards its shaft end.

11. Joint prosthesis according to one of the preceding claims **characterised in that** the joint connecting element is formed as a bone screw (3.1) or as a ball joint (3.2).

12. Joint prosthesis according to claim 11 **characterised in that** the ball joint can be fitted together with the head part (3) by a conical push-fit connection.

13. Structural kit for producing a joint prosthesis according to claim 1 or 2 **characterised by** a selection of head parts (3) of different length and/or different diameter as well as a selection of shaft parts (2) of different length, different diameter or different curvature.

14. Structural kit according to claim 13 **characterised in that** for the head parts (3) a diameter is proposed in the region of 12 to 17 mm and for the shaft parts (2) a length is proposed in the region of 200 to 320 mm and a diameter in the region of 10 to 14 mm.

## Revendications

1. Prothèse articulaire (1), comprenant une partie de fût (2), susceptible d'être mise en place dans un os long et comportant un perçage longitudinal (4), et une partie de tête (3) qui s'y raccorde de façon proximale, avec un élément de raccord articulaire (3.1, 3.2) monté en translation longitudinale dans cette partie de tête, les axes longitudinaux de la partie de fût (2) et de la partie de raccord articulaire (3.1, 3.2) formant un angle obtus, dit angle d'inversion, et la partie de tête (3) comporte une zone en forme de douille, qui présente un perçage traversant incliné sensiblement sous l'angle d'inversion par rapport à l'axe du fût (11) et adapté à la section transversale du fût (20) de l'élément de raccord articulaire (3.1, 3.2) susceptible d'être enfilé dans ce perçage, **caractérisée en ce que** la partie de tête (3) présente un perçage axial (5) qui s'étend en direction de la partie de fût (2) et qui traverse l'élément de raccord articulaire (3.1, 3.2) monté dans la partie de tête (3).

2. Prothèse articulaire selon la revendication 1, **caractérisée en ce que** le perçage (5) est pourvu d'un pas de vis pour un organe de vissage servant à détacher la jonction d'enfichage avec la partie de fût respective suivante, le diamètre (D1) du perçage (5) étant supérieur au diamètre (D2) d'un perçage (4) ménagé dans la partie de fût suivante.

3. Prothèse articulaire selon l'une ou l'autre des revendications 1 et 2, **caractérisée en ce que** le rapport entre le diamètre (D1) du perçage (5) dans la partie de tête (3) et le diamètre (D2) du perçage dans la partie de fût suivante a une valeur dans la plage de 1,5 à 2,5.

4. Prothèse articulaire selon l'une ou l'autre des revendications 2 et 3, **caractérisé en ce que** le pas de vis dans le perçage (5) ménagé dans la partie de tête (3) ou dans la partie de fût (2) qui précède en direction proximale s'étend sur la totalité de sa longueur.

5. Prothèse articulaire selon l'une des revendications précédentes, **caractérisée en ce que** le perçage (4) prévu dans la partie de fût (2) suivante n'est pourvu d'un pas de vis que dans la région de son extrémité proximale (6).

6. Prothèse articulaire selon la revendication 5, **caractérisée en ce que** l'extrémité proximale du perçage à pas de vis (6) de la partie de fût (2) suivante comporte un chanfrein (11).

7. Prothèse articulaire selon l'une ou l'autre des revendications 1 et 2, **caractérisée en ce que** l'élément de raccord articulaire (3.1, 3.2) est monté de manière à pouvoir être arrêté avec son fût (30) dans la région en forme de douille de la partie de tête (3).

8. Prothèse articulaire selon la revendication 7, **caractérisée en ce que** le fût (20) de l'élément de raccord articulaire (3.1, 3.2), qui traverse la partie de tête (3), est susceptible d'être expansé par des moyens d'écartement en vue de son arrêt.

9. Prothèse articulaire selon la revendication 8, **caractérisée en ce que** le fût (20) est réalisé au moins partiellement sous la forme d'une douille susceptible d'être écartée, dotée de fentes (13) qui s'étendent en direction longitudinale.

10. Prothèse articulaire selon la revendication 9, **caractérisée en ce que**, pour l'écartement de la région fendue de l'élément de raccord articulaire (3.1, 3.2), il est prévu un goujon vissé avec une zone qui va en se rétrécissant vers l'extrémité de son fût.

11. Prothèse articulaire selon l'une des revendications précédentes, **caractérisée en ce que** l'élément de raccord articulaire est réalisé sous la forme de vis à os (3.1) ou sous la forme de bille articulaire (3.2).

12. Prothèse articulaire selon la revendication 11, **caractérisée en ce que** la bille articulaire est susceptible d'être assemblée avec la partie de tête (3) au moyen d'une liaison à enfichage conique.

13. Jeu de composants pour réaliser une prothèse articulaire selon l'une ou l'autre des revendications 1 et 2, **caractérisé par** une série de parties de tête (3) de différentes longueurs et/ou de différents diamètres, ainsi qu'une série de parties de fût (2) de longueurs différentes, de diamètres différents ou de courbures différentes.

14. Jeu de composants selon la revendication 13, **caractérisé en ce que** l'on prévoit pour les parties de tête (3) un diamètre dans la plage de 12 à 17 mm, et pour les parties de fût (2) une plage de longueurs de 200 à 320 mm et une plage en diamètre de 10 à 14 mm.
